## Europäisches Patentamt
## European Patent Office
## Office européen des brevets

(19)

(11) Veröffentlichungsnummer: **0 066 545**
**B1**

(12) **EUROPÄISCHE PATENTSCHRIFT**

(45) Veröffentlichungstag der Patentschrift:
**12.03.86**

(21) Anmeldenummer: **82810229.3**

(22) Anmeldetag: **26.05.82**

(51) Int. Cl.⁴: **A 41 B 13/02**

(54) **Windel, insbesondere Höschenwindel, und Verfahren zu ihrer Herstellung.**

(30) Priorität: **26.05.81 CH 3451/81**

(43) Veröffentlichungstag der Anmeldung:
**08.12.82 Patentblatt 82/49**

(45) Bekanntmachung des Hinweises auf die Patenterteilung:
**12.03.86 Patentblatt 86/11**

(84) Benannte Vertragsstaaten:
**AT CH DE FR GB IT LI NL SE**

(56) Entgegenhaltungen:
**EP - A - 0 000 969**
**EP - A - 0 017 770**
**EP - A - 0 021 662**
**DE - A - 3 016 197**
**FR - A - 2 305 944**
**GB - A - 2 010 682**
**GB - A - 2 033 210**
**US - A - 3 758 363**

(73) Patentinhaber: **Curt G. Joa, Incorporated, 1500 No. High Ridge Road, Boynton Beach, FL 33425 (US)**

(72) Erfinder: **Joa, Curt G., 1500 No. High Ridge Road, Boynton Beach, FL 33435 (US)**

(74) Vertreter: **Nikolaiski, Eckhard Siegfried, Dr., Postfach 16 Auwiesenstrasse 2, CH-8352 Räterschen (CH)**

## Beschreibung

Die Erfindung betrifft eine Windel, insbesondere Höschenwindel, mit einer flüssigkeitsundurchlässigen Aussenschicht, einer flüssigkeitsdurchlässigen Innenschicht und einer saugfähigen Zwischenlage, mit einem vorderen Windelrand, einem hinteren Windelrand und zwei beide Ränder verbindenden Seitenrändern, wobei jeder Seitenrand in seinem Mittelbereich eine mindestens angenähert trapezförmige Randaussparung aufweist, deren kürzere parallele Trapezseite innen liegt, und mit einem elastischen Abdichtungsband, das längs jeden Seitenrandes in der Windel mindestens an seinen beiden Enden befestigt ist. Weiterhin betrifft die Erfindung eine Vorrichtung zur Herstellung von Windeln, insbesondere Höschenwindeln, mit einer Station zum Aufbringen elastischer kontinuierlicher Abdichtstreifen auf die Aussenränder einer Bahn des Windelmaterials, bei der eine Trommel an ihrem äusseren Umfang aufeinanderfolgend mit Abstand voneinander Zwischenräume aufweist und eine Fördereinrichtung mit Auslenkorganen für eine Aussenschichtbahn für die Windel vorgesehen ist, deren Förderweg denjenigen des äusseren Umfangs der Trommel in den Zwischenräumen zweimal kreuzt, und die Förderwege des äusseren Umfangs und der Fördereinrichtung zwischen Kreuzungsstellen mindestens auf einem Teil parallel verlaufen und eine Zuführ- und Aufbringeinrichtung für die Abdichtstreifen in den Förderweg der Trägerabschnitte vorgesehen ist, der eine in die Zwischenräume eingreifende Trenneinrichtung für die Abdichtbänder nachgeordnet ist.

Es ist bereits eine Inkontinenz-Hose bekannt (EP-PS Nr. 969), bei welcher die Enden von zwei elastischen Bändern längs der beiden Seitenränder entweder versteift ausgeführt oder an der Hose nicht befestigt sind, damit die Enden die Hose zusammenziehen können. Hierzu ist erforderlich, dass die elastischen Bänder eine ihrer Kontrahierfähigkeit entsprechend ausreichende Länge aufweisen, um bei der bekannten Hose einen gewünschten zusammengefalteten Teil zu erhalten. Die bekannte Hose ist wegen der gegenüber Kleinkindern anderen Anatomie der Beine bei Erwachsenen nur für letztere Personen geeignet.

Eine bekannte Vorrichtung zur Herstellung von Windeln der eingangs genannten Art (z.B. EP-PS 17 770) besitzt am äusseren Umfang einer Walze Nuten, in welche Stangen eingreifen. Die Stangen werden durch eine Förderkette geführt, welche in üblicher Weise um ein Kettenrad gelegt ist, so dass die Förderkette parallel zum Umfang der Walze läuft.

Es ist die Aufgabe der vorliegenden Erfindung, eine Windel der eingangs genannten Art zu schaffen, bei welcher die Elastizität der Abdichtbänder voll ausgenutzt werden soll, sowie mit einer Vorrichtung der eingangs genannten Art eine erforderliche Länge der Abdichtbänder mit einem Minimum an Abfall zu erreichen.

Diese Aufgabe wird für die Windel der eingangs genannten Art erfindungsgemäss dadurch erreicht, dass jedes der befestigten Enden des Abdichtungsbandes in ungespanntem Zustand desselben der in Endstellung untersten Querlinie näherliegt als dem benachbarten vorderen bzw. hinteren Rand der Windel und dass die Länge jeden Abdichtungsbandes der Länge der zugeordneten kürzeren Trapezseite entspricht.

Für die Vorrichtung der eingangs genannten Art wird die Aufgabe erfindungsgemäss dadurch gelöst, dass am äusseren Umfang der Trommel Trägerplatten für die Aussenschichtbahn mit Abstand voneinander angeordnet sind, die Auslenkorgane durch im Innern der Trommel liegende Elemente parallel zum Trommelumfang führbar sind und die Fördereinrichtung zwischen den Kreuzungsstellen auf einer gekrümmten Unterlage aufliegt, durch welche die Länge der Fördereinrichtung verkürzt ist, und die Einrichtungen zur Zuführung der Abdichtstreifen in gestrecktem Zustand zur Trommel vorgesehen sind.

Die Erfindung geht von der Erkenntnis aus, dass die Endabschnitte aller bisher verwendeten Abdichtungsbänder bei der Verwendung der Windel praktisch ungedehnt bleiben und daher funktionell unwirksam sind. Da die Kosten für die üblicherweise durch gewobenes Gummiband gebildeten Abdichtungsbänder einer Windel ausserordentlich hoch sind, lassen sich die Herstellungskosten der Windel beim Fortlassen der funktionell unwirksamen Endabschnitte beträchtlich verringern. Ausserdem erlaubt es ein derart verkürztes Abdichtungsband

a) dass bei der Herstellung der Windel auf apparativ einfachste Weise eine bessere Anpassung an die Anatomie des menschlichen Körpers erzielbar ist, was bei Strampel-, Geh- und Laufbewegungen besonders wichtig ist; und
b) dass beim Tragen einer solchen Windel unter im übrigen gleichbleibenden Bedingungen eine bessere Saugfähigkeit und Abdichtung im besonders kritischen Bereich erzielt wird.

Eine besonders vorteilhafte Ausgestaltung der Windel nach der Erfindung kann mit den Merkmalen des Anspruches 2 erreicht werden. Vorteilhafte Ausführungsformen der Vorrichtung nach der Erfindung sind durch Massnahmen der Ansprüche 4–10 beschrieben.

Bevorzugte Ausführungsformen der Erfindung sind nachfolgend in Ausführungsbeispielen anhand der Zeichnungen näher erläutert. Es zeigen:

Fig. 1 eine Windel in schematischer Darstellung in Draufsicht;
Fig. 1a die Windel von Fig. 1 in einer Seitenansicht;
Fig. 2 die Windel der Fig. 1 in Endstellung in schaubildlicher Darstellung;
Fig. 3 eine aus Windeln gem. Fig. 1 gebildete Windelbahn mit gespannten Abdichtungsbändern, aus der nach dem Durchtrennen in den Pfeilrichtungen jeweils einzelne Windeln gem.

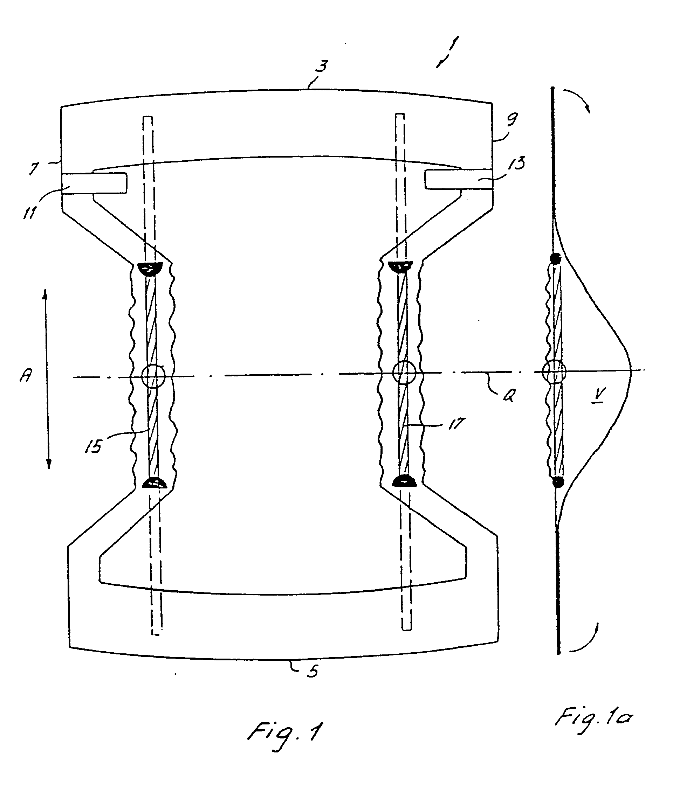

Fig. 1 gebildet werden;

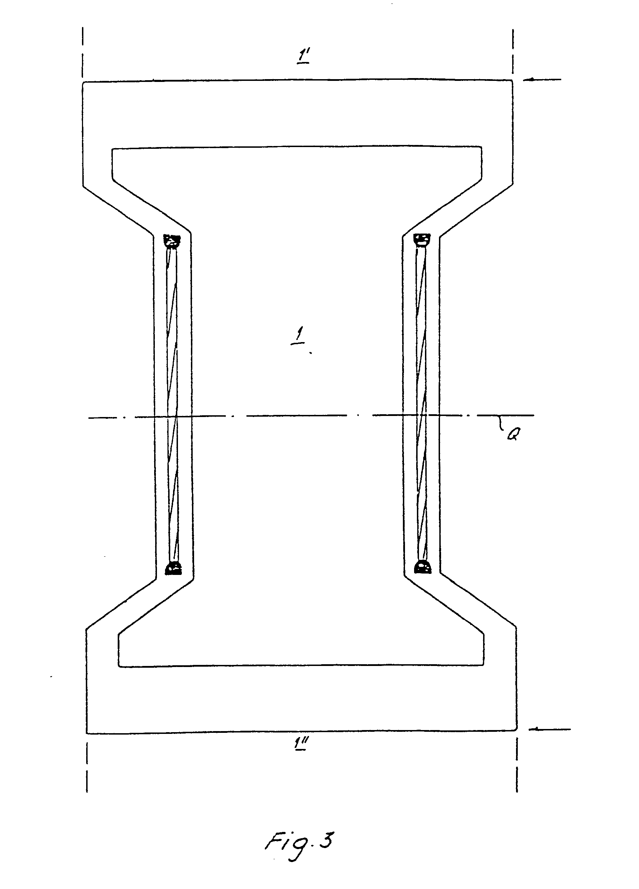

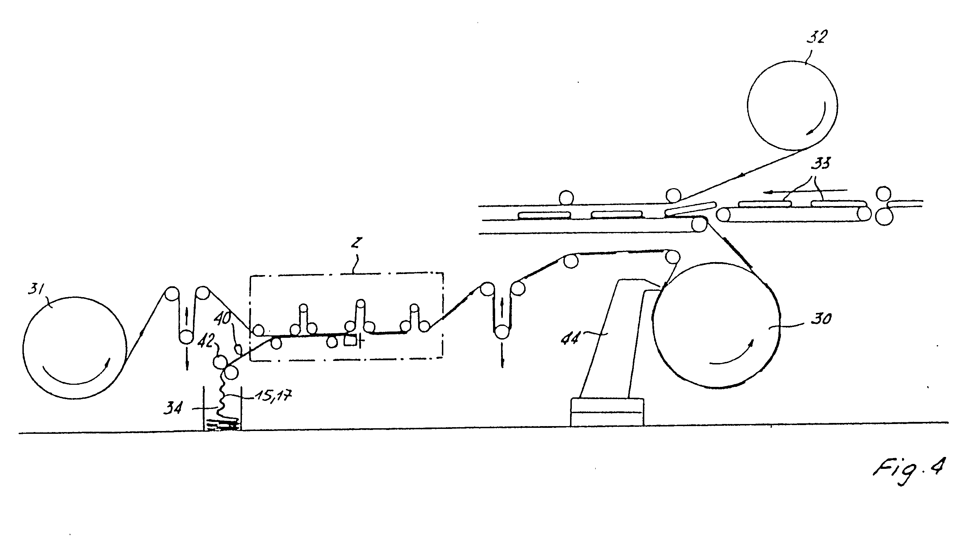

Fig. 4 eine Vorrichtung für die Herstellung der Windelbahn der Fig. 3 in schematischer Darstellung in einer Ansicht;

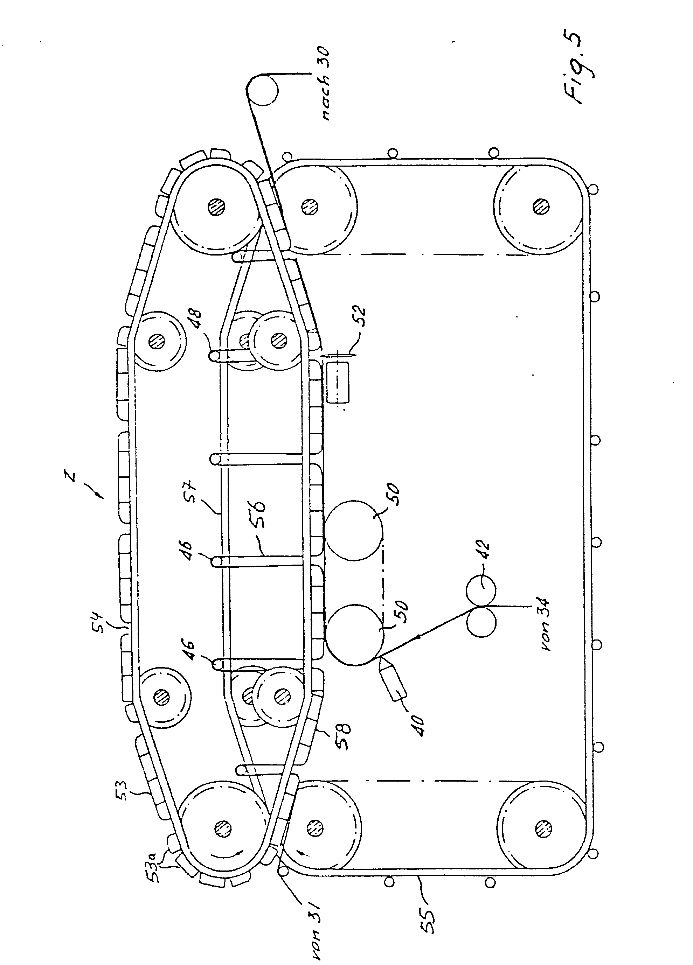

Fig. 5 einen Teil der Vorrichtung von Fig. 4 in vergrösserter schematischer Darstellung; und

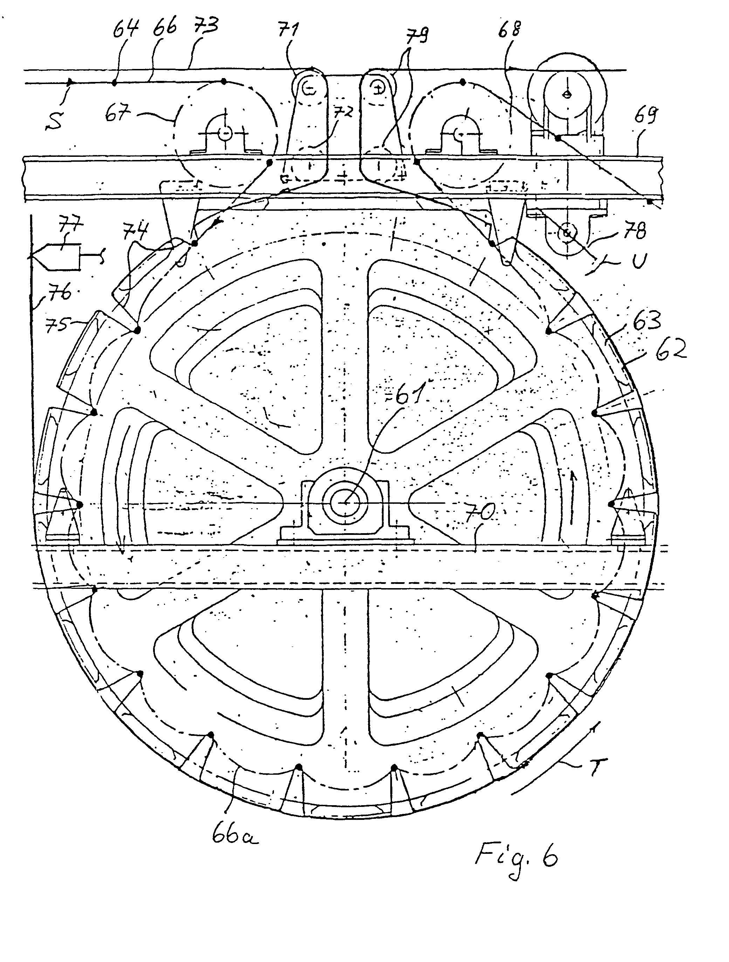

Fig. 6 eine andere Ausführungsform eines Teils einer Vorrichtung zur Herstellung der Windelbahn in schematischer Darstellung.

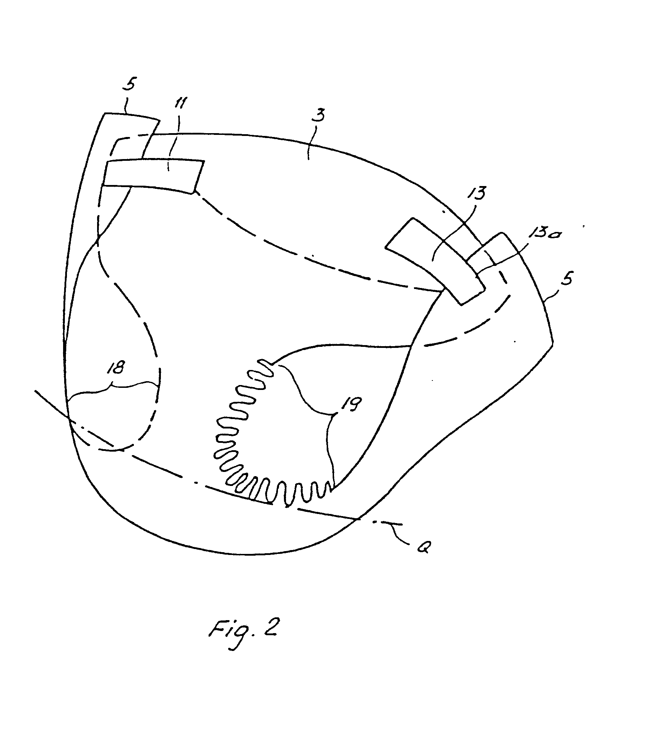

Die Windel 1 der Fig. 1 weist in an sich bekannter Weise eine flüssigkeitsundurchlässige Aussenschicht, z.B. aus Polyäthylen, und eine flüssigkeitsdurchlässige Innenschicht auf, die mindestens längs der Seitenränder 7, 9 miteinander verbunden sind, und zwischen denen sich eine saugfähige Zwischenlage befindet. Ferner weist die Windel 1 in Fig. 1 Mittel auf, z.B. zwei zweiflügelige Selbstklebestreifen 11, 13, um den in Fig. 1 oberen Vorderteil der Windel 1 nach dem Anlegen an den menschlichen Körper mit dem in Fig. 1 unteren Rückenteil der Windel 1 zu verbinden und so das Verrutschen der Windel zu verhindern und eine Art Windelhose zu bilden (Fig. 2). Auf die Ausbildung, Anordnung und Anzahl dieser Mittel und auf deren Anwesenheit kommt es hier jedoch nicht an; die Windel kann auch durch Knöpfe, Bänder oder sonstwie in ihrer Endstellung (Fig. 2) unverrutschbar gehalten werden. Die Zwischenlage ist zweckmässig als ein lose zusammengehaltenes Saugkissen 33 (Fig. 4) ausgebildet.

Bei der Windel 1 der Fig. 1 ist die strichpunktierte, in Fig. 2 unterste Querlinie Q eine Symmetrielinie, so dass der Bauchteil und der Rückenteil der Windel 1 zur Erhöhung der Narrensicherheit bei der Anwendung und gleichzeitig zur Vereinfachung der Herstellung vertauschbar sind. Die Windel weist in der Darstellung der Fig. 1 einen oberen Windelrand 3 und einen unteren Windelrand 5 sowie die zwei gleichlangen, letztere miteinander verbindende Seitenränder 7, 9 auf, die in ihrem Mittelabschnitt zur Anpassung an die Anatomie des menschlichen Körpers je eine mindestens angenähert trapezförmige Randaussparung aufweisen. Die jeweils kürzeren der beiden parallelen Trapezseiten sind einander zugekehrt und aus den weiter unten erläuterten Gründen gerüscht.

Die obligatorischen Befestigungsstellen von zwei elastischen Abdichtungsbändern 15, 17 an der Aussenschicht der Windel 1 sind in Fig. 1 durch vier voll ausgefüllte Halbkreise und zwei fakultative Befestigungsstellen sind durch zwei Hohlkreise dargestellt.

Wesentlich ist hier, dass bei der Windel 1 der Fig. 1 die Länge A der beiden im Windelinnenraum vorhandenen, längs jedes Windelseitenrands 7, 9 angeordneten, elastischen Abdichtungsbänder 15 und 17 zur besseren Anpassung an die Anatomie des menschlichen Körpers im ungespannten Zustand jeweils höchstens halb so lang sind wie der Abstand des vorderen Windelrands 3 vom hinteren Windelrande 5. Funktionell unwirksame Endabschnitte des Standes der Technik, wie sie beispielsweise durch die unterbrochenen Linien in Verlängerung der Abdichtungsbänder 15, 17 dargestellt sind, fehlen hier. Jedes Abdichtungsband 15, 17 ist vorzugsweise mindestens an seinen beiden Enden im Innern der Windel 1 derart befestigt, dass im nicht gespannten, d.h. ungespannten Zustand der Abdichtungsbänder 15, 17 die Innenschicht der Windel eine Vorwölbung V (in Fig. 1a übertrieben gross dargestellt) bildet. Diese ist dem Schrittbereich des menschlichen Körpers besonders angepasst und schafft einen genügend grossen Raum, um den überwiegenden Teil des Saugkissens 33 (Fig. 4) aufzunehmen. Vor dem Anlegen einer solchen Windel 1 ist natürlich darauf zu achten, dass die flüssigkeitsundurchlässige Aussenschicht der Windel 1 aussen, d.h. in Fig. 1a gesehen links liegt. Dazu wird die Windel zweckmässig so gelagert und verpackt, dass ihre obere und ihre untere Hälfte im Sinne der beiden gekrümmten Pfeile der Fig. 1a aufeinander zu geklappt sind.

Die gerüschten Bereiche 18, 19 in Fig. 2 lassen erkennen, dass

a) die Dehnung der Abdichtungsbänder 15, 17 im Bereich der Querlinie Q am grössten ist;

b) ihre Abdichtung gegen die betreffenden Oberschenkel daher dort am besten ist; und

c) die Verdickung des benachbarten Saugkissenbereichs und daher dessen Saugfähigkeit dort am grössten sind. Nach dem Anlegen der Windel 1 entsteht so zwischen beiden gerüschten Bereichen 18, 19 in sehr erwünschter Weise eine Flüssigkeitsauffangmulde (unten in Fig. 2).

In Fig. 1 besteht die Besonderheit darin, dass jedes Ende des Abdichtungsbands 15, 17 im nicht gespannten Zustand der in Endstellung untersten Querlinie Q näher ist als dem benachbarten vorderen bzw. hinteren Rande 3 bzw. 5 der Windel 1. Die Länge A jedes Abdichtungsbands 15, 17 entspricht im nichtgespannten Zustande der Länge der zugeordneten kürzeren Trapezseite und beträgt je nach der Grösse der Windel z.B. nur noch 6 bis 30 cm. Gleichzeitig wird dadurch die Windel 1 in anatomisch sehr erwünschter Weise verformt, und vor allem wird die unmittelbare Umgebung jedes Abdichtungsbands 15, 17 im wichtigsten Bereich der Windel 1 faltenbalgartig gerüscht. Gleichzeitig wird dort die Dicke des Saugkissens 33 (Fig. 4) in sehr erwünschter Weise vergrössert.

Die Massenproduktion der Windel 1 der Fig. 1 lässt sich auf wirtschaftlichste und apparativ einfachste Weise und mit einem Mindestmass an Abfall durchführen, wenn man, wie Fig. 3 zeigt,

a) zuerst eine endlose Windelbahn 1', 1, 1'' herstellt, wobei die Abdichtungsbänder 15, 17 durch eine in Längsrichtung ausgeübte Zugkraft gespannt werden, die für die Vorbewegung der Bahn ohnehin irgendwie aufgebracht werden muss, und

b) die Windelbahn 1', 1, 1'' in Richtung der in Fig.

3 horizontal dargestellten Pfeile durchtrennt, wonach die einzelnen Windeln 1', 1, 1" wegen des dabei auftretenden Spannungsverlustes der Abdichtungsbänder 15, 17 von selbst die in Fig. 1 dargestellte Form annehmen.

Dabei geht man zweckmässig so vor, dass man zur Herstellung der Windelbahn 1', 1, 1" zunächst eine kontinuierlich geförderte Bahn der Aussenschicht in mehrere Bahnabschnitte unterteilt, indem die Bahn schlaufenförmig herausgezogen wird und die Bahnabschnitte gleichzeitig und zusammen mit den dazwischen befindlichen Bahnschlaufen kontinuierlich und synchron weiterbefördert werden. Auf die Aussenränder der Bahnabschnitte wird ein z.B. mit Bindemittel versehenes, gestrecktes bzw. gespanntes, elastisches Abdichtband kontinuierlich aufgebracht und unter Andrücken auf der Bahn befestigt. Die den Abstand zwischen den Bahnabschnitten überbrückenden Abdichtbänder werden dann zwischen zwei Bahnabschnitten durchtrennt, so dass nach Auflösen der Schlaufen das elastische Abdichtband lediglich auf den zuvor gebildeten Bahnabschnitten vorliegt. Zwischen der endlosen Bahn der Aussenschicht und einer endlosen Bahn der Innenschicht werden dann abgelängte Abschnitte der saugfähigen Zwischenlage im Abstand voneinander positioniert und beide endlose Bahnen an den den Aussenrändern der fertigen Windeln entsprechenden Stellen mittels des Bindemittels, vorzugsweise unter Aneinanderdrükken, miteinander verbunden.

Im oberen Teil der Fig. 4 ist dargestellt, dass die Windelbahn im Prinzip wie eine dreischichtige Verbundstoffbahn hergestellt wird. Dazu sind eine Vorratsrolle 31 für die Aussenschichtbahn, ein Vorrat 34 für zwei endlose Abdichtungsbänder 15, 17 (nur eines gezeigt), eine Vorratsrolle 32 für die Innenschichtbahn sowie eine Vorrichtung erforderlich, um eine Folge von Saugkissen 33 im Abstand voneinander zuzuführen. Dabei soll jedes Saugkissen 33 genau und reproduzierbar von einem nicht dargestellten Saugkissenvorrat abgetrennt und in den Raum zwischen dem vorderen Rand 3 und dem hinteren Rand 5 der Windel 1 genau positioniert sein.

Das Hauptproblem besteht jedoch darin, die beiden Abdichtungsbänder 15, 17 im Inneren jeder Windel beidenends so zu befestigen, dass die Vorwölbung V der Fig. 1a entsteht. Der linke Teil der Fig. 4 und die Fig. 5 zeigen, dass mit diesem Arbeitsschritt begonnen wird. Die beiden aus dem Vorrat 34 entnommenen, endlosen Abdichtungsbänder durchlaufen zuerst den Spalt eines Walzenpaars 42, den sie geradlinig verlassen. Danach wird durch eine erste Bindemittelauftragevorrichtung 40 Bindemittel punktweise auf die vorgesehenen Stellen der beiden endlosen Abdichtungsbänder 15, 17 aufgetragen. Gleichzeitig wird die endlose Bahn der Aussenschicht vom Vorrat 31 abgezogen und läuft längs des unteren Trums eines Endlosförderers Z, der, wie insbesondere Fig. 5 zeigt, biegbare, aus Einzelelementen 53a aufgebaute Trägerabschnitte 53 für die Bahn 31 besitzt, die mit Abstand aufeinanderfolgen, d.h. zwischen denen Zwischenräume 54 vorliegen. Dabei ist es besonders wichtig, dass man zur Erzielung der Vorwölbung V (Fig. 1a) beim Befestigen der beiden Abdichtungsbänder 15, 17 die Länge der endlosen Bahn 31 der Aussenschicht durch Schlaufenbildung um einen bestimmten Betrag scheinbar vergrössert. Gleichzeitig wird so auch der in Fig. 1 und 3 erkennbare Abstand der Abdichtungsbänder 15, 17 vom vorderen 3 und hinteren Windelrand 5 sichergestellt.

Fig. 5 zeigt die Schlaufenbildung der endlosen Bahn der Aussenschicht durch Umlenkwalzen 46, 48. Diese Umlenkwalzen 46, 48, die auch als Stangen vorliegen können, werden durch ein kontinuierlich bewegtes Förderband 55 jeweils durch die Freiräume 54 hindurch in das Innere des Förderers Z bewegt, d.h. die Umlenkwalzen 54 kommen in Eingriff mit den Zwischenräumen 54 und kreuzen dabei den Förderweg der Fördereinrichtung. Dabei ergreift jeweils eine Umlenkwalze 46 die Bahn 31 ausserhalb des Endlosförderers Z, zieht eine Schlaufe 56 aus der Bahn 31 nach innen durch den Freiraum 54 hindurch und bewegt sich und die Schlaufe entlang des oberen Trums 57 des Förderbandes 55 synchron zu den Trägerabschnitten 53. Die dem unteren Trum des Endlosförderers Z zugeführten Abdichtbänder 15, 17 können daher nur mit Bahnabschnitten 58 in Berührung kommen, die sich auf den Trägerabschnitten 53 befinden, und werden daher nur auf diesen Bahnabschnitten 58 befestigt. Die Abdichtbänder 15, 17 sind in gestrecktem bzw. in gespanntem Zustand, was wie vorstehend erwähnt, durch eine in Förderrichtung angreifende Zugkraft erfolgen kann, die z.B. vom Walzenpaar 42 weg durch die Vorbewegung der Bahn 31 aufgebracht sein kann. Die Befestigung der beiden endlosen Abdichtungsbänder 15, 17 erfolgt an den Berührungsstellen der beiden Andruckwalzen 50. Die Umlenkwalze 48 hat die zusätzliche Funktion, dass sie die endlose Bahn 31 der Aussenschicht von den beiden endlosen Abdichtungsbändern fernhält, wenn letztere mittels einer Trennvorrichtung 52 durchtrennt werden. Durch die Schlaufenbildung zwischen den Trägerabschnitten 53 bzw. Bahnabschnitten 58 können durch die Trennvorrichtung 52 daher nur die Abdichtbänder 15, 17 durchtrennt werden. Auf diese Weise sind dann nachfolgend nur die Bahnabschnitte 58 an den Aussenrändern mit den Abdichtungsbändern versehen, wobei die Bahnabschnitte 58 dann dem Teil der fertigen Windel 1 entsprechen, welcher sich im entspannten Zustand der Abdichtbänder 15, 17 faltenbalgartig rüscht. Auf diese Weise entstehen also die in Fig. 1 und 1a schraffiert dargestellten Abschnitte, d.h. der Abdichtungsbänder 15, 17. Nach dem Befestigen der Abdichtungsbänder 15, 17 wird die Schlaufenbildung 56, d.h. die scheinbare Verlängerung der Bahn 31 wieder aufgehoben. Dieses geschieht wiederum unter Kreuzen der Förderwege der Fördereinrichtung Z und der Umlenkwalzen 46, 48, die dabei wiederum jedoch in anderer Richtung durch die Zwischenräume 54 hindurchtreten und die Bahn 31 freigeben. Dabei bleiben jedoch die Abdichtungsbänder 15, 17 im gespannten Zustand mit den abschnittsweise befestigten Abdichtungsbändern 15, 17. Das Gebilde wird dann um eine Führungswalze 30 herumgeführt, wobei eine zweite Bindemittelauftragevorrichtung 44 auf die den Aussenrändern der fertigen Windeln entsprechenden Stellen der endlosen Bahn der Aussenschicht Bindemittel aufträgt.

Der rechte Teil der Fig. 4 zeigt, wie sich die Bahn 31 mit der endlosen Bahn der Innenschicht vereinigt, die vom Vorrat 32 abgezogen wird. Vor dieser Vereinigung werden jedoch zwischen beiden Bahnen die vorgängig von einer dritten Bahn abgelängten Saugkissen 33 im Abstand voneinander genau positioniert. Es ist von Vorteil, wenn man zum Positionieren der Saugkissen 33 im Abstand voneinander einen intermittierend arbeitenden Bandförderer verwendet. Werden dann die Innenschichtbahn 32 und die Aussenschichtbahn 31 an den den Aussenrändern der fertigen Windeln entsprechenden Stellen mittels des Bindemittels miteinander verbunden, vorzugsweise unter örtlichem Aneinanderdrücken, so ist die Windelbahn 1', 1, 1" (Fig. 3) fertig. Sie kann auf einen Kern aufgewickelt oder sofort in einzelne Windeln unterteilt werden, von denen jede einzelne nach dem Abtrennen die in Fig. 1 und 1a dargestellte Form, d.h. unter Entspannung der elastischen Abdichtbänder 15, 17 gerüschte Form annimmt.

Vorzugsweise erfolgt die Ausbildung von mindestens zwei Bahnabschnitten mittels der Schlaufenbildung zwischen denselben, wobei mindestens zwei Schlaufen gebildet werden. Das Abdichtband kann mittels eines geeigneten Klebstoffes mit der Bahn der Aussenschicht verbunden werden. Es ist aber auch möglich, das Bindemittel z.B. auf der Aussenschichtbahn vorzusehen. Das Abdichtband kann aber auch bei entsprechender Gestaltung seines Materials und/oder desjenigen der Aussenschicht mit der letzteren verschweisst werden. Anstatt der beiden Andruckwalzen 50 (Fig. 5) lässt sich z.B. auch ein endlos geführter Andruckriemen mit V-Profil verwenden, was in Fig. 5 durch eine strichpunktierte Linie angedeutet ist. Die Befestigung der Abdichtungsbänder 15,17 erfolgt vorzugsweise durch Heissiegelung («Hot Melt»). Schliesslich ist zu betonen, dass die unterste Querlinie Q keine Symmetrielinie der Windel 1 zu sein braucht. Das Kreuzen der Fördereinrichtung Z und der Umlenkwalzen 46, 48 durch die Zwischenräume 54 kann durch entsprechende Abstimmung des Antriebs für die Fördereinrichtung Z und das Förderband 55 erfolgen.

Bei der Ausführungsform der Fig. 6 ist eine Trommel 60 mit einer Welle 61 in Richtung von Pfeil T drehbar und antreibbar gelagert und besitzt an ihrem äusseren Umfang 62 Tragplatten 63. Letztere sind um den Umfang 62 gesehen derart mit Abstand voneinander angeordnet, dass bei Drehung der Trommel 60 Stangen 64 durch die Zwischenräume oder Spalte 65 hindurchtreten können, die mittels eines Förderelementes 66, vorzugsweise einer Förderkette in Pfeilrichtung S zugeführt werden. Die parallel zu den Tragplatten 63 liegenden Stangen 64 kreuzen dabei die Trommeloberfläche und dringen durch die Spalte 65 in das Innere der Trommel 60 ein. Dort werden die Stangen 64 durch nicht dargestellte Elemente parallel zum Trommelumfang mittels des Förderelementes 66 und mit entsprechender Geschwindigkeit geführt, so dass sie zusammen mit dem Trommelumfang mitwandern können. Nach einer Trommelumdrehung von mehr als 180° treten die Stangen 64 wieder aus der Trommel 60 durch die Spalten 65 aus, wobei sie den Trommelumfang 62 wiederum kreuzen. Die Zuführung und die Wegführung der Förderkette 66 erfolgt über eine Führungsrolle 67 bzw. 68, die drehbar an einem Gestellteil 69 gelagert sind. Durch nichtdargestellte Mittel wird die Förderkette wieder zur Führungsrolle 67 zurückgeführt. Obwohl nur eine Förderkette gezeigt ist, werden die Stangen 64 beidseitig von je einer Förderkette 6 getragen. Die Lagerung der Welle 61 und damit der Trommel 60 erfolgt an einem Gestellteil 70.

Über Zuführrollen 71, 72 wird eine Bahn 73 einer Aussenschicht für die Windel zugeführt und gelangt auf die Trommel 60. Beim Durchtritt einer Stange 64 durch den Spalt 65 zwischen zwei Tragplatten 63 von aussen nach innen wird eine Bahnschlaufe 74 gebildet, was sich durch Drehung der Trommel 60 und fortlaufende Zuführung von Stangen 64 am Umfang 62 der Trommel 60 fortsetzt. Infolge der Auslenkung der Bahn 73 unter der Schlaufenbildung 74 aus dem Förderweg am Umfang 62 der Trommel 60 umspannt die Bahn 73 die Trommel 60 und damit die Tragplatten 63. Dadurch werden auf den Tragplatten 63 Bahnabschnitte gebildet, die durch die Bahnschlaufen 74 unterbrochen sind. Durch entsprechende Abstimmung von Umfangsgeschwindigkeit der Trommel 60 und Fördergeschwindigkeit der Förderkette 66 und damit der Stangen 64 werden die Bahnabschnitte und auch die Schlaufen 74 zusammen und synchron gefördert. Den äusseren Rändern der Bahnabschnitte 75 auf den Tragplatten 63 werden Abdichtbänder bzw. -streifen 76 tangential zugeführt (nur ein Abdichtband 76 gezeigt), die mittels einer Einrichtung 77 an ihrer den Bahnabschnitten zugewandten Seite vor der Berührung mit den Bahnabschnitten 75 mit einem Bindemittel beschichtet werden. Da am äusseren Umfang 62 nur die Bahnabschnitte 75 auf den Tragplatten 63 den Abdichtbändern 76 angeboten werden, können die letzteren auch nur mit diesen verbunden werden. Die Bahnschlaufen 74 bleiben dagegen frei vom Abdichtband 76. Die Abdichtbänder 76 bestehen aus einem elastischen Material und werden in ihrem gestreckten bzw. gespannten Zustand zugeführt, was z.B. in ähnlicher Weise wie die in der Ausführungsform nach Fig. 4 und 5 beschrieben erfolgen kann. Nach einem Umlauf von mehr als 180° gelangen die an der äusseren Oberfläche liegenden Abdichtbänder 76 in den Bereich eines in Pfeilrichtung U rotierenden Messers 78, das derart angeordnet und in Drehung versetzt wird, dass es in Eingriff mit einem Spalt 65 zwischen zwei Tragplatten 64 gelangt. Dabei werden die Abdichtbänder 76 durchgetrennt, so dass sie als Bandabschnitte nur auf den Bahnabschnitten 75 vorliegen. Kurz danach treten die Stangen unter Auflösen der Bahnschlaufen 74 durch die Spalte 65 wieder aus. Die Bahn 73 mit Abschnitten der Abdichtbänder 76 wird nachfolgend über Abführrollen 79 abgeführt und kann danach z.B. wie in der Ausführungsform nach Fig. 4 und 5 beschrieben weiterbehandelt und zur Windel 1 aufgearbeitet werden.

In gleicher Weise wie bei der Ausführungsform nach Fig. 4 und 5 beschrieben liegt hierbei der Vorteil vor, dass von der Bahn 73 der Aussenschicht lediglich diejenigen Bahnabschnitte 75 mit Abdichtband 76 versehen werden, welche später nach Fertigstellung der Windel den gerüschten Teil und damit bei z.B. der Windel 1 nach Fig. 1 die Vorwölbung V ergeben.

Wie bereits erwähnt, dringt das Messer 78 im Bereich der Schlaufen lediglich durch die elastischen Abdichtbänder 76, so dass die Bahn 73 unzertrennt bleibt. Das z.B. zweiflügelig ausgebildete Messer kann z.B.. mit je einer halben Umdrehung einmal in einen der Spalte 65 eindringen und die Abdichtbänder 76 durchtrennen. In vorteilhafter Weise werden die Messer durch einen kleinen Motor mit 1800 U/min angetrieben. Selbstverständlich ist für jeden Rand der Bahn 73, d.h. für jedes Abdichtband, ein rotierendes Messer vorgesehen. Um z.B. kurze Schnittenden der elastischen Abdichtbänder 76 zu erzielen, wird der Abstand zwischen den Tragplatten 63 vorzugsweise entsprechend klein gewählt. Dies kann dadurch erreicht werden, dass man z.B. die Förderkette 66 für die Stangen 74 über gekrümmte Bahnsegmente vorverkürzt, wie es durch die gekrümmte Führung 66a der Förderketten 66 im Innern der Trommel 60 angedeutet ist. Dadurch können die Umlenkstangen 64 mit einem kleinen Spiel durch die Spalte 65 hindurchtreten, so dass die elastischen Bänder 76 mit einem Minimum an Abfall von unverklebten Enden durchtrennt werden können.

## Patentansprüche

1. Windel, insbesondere Höschenwindel, mit einer flüssigkeitsundurchlässigen Aussenschicht, einer flüssigkeitsdurchlässigen Innenschicht und einer saugfähigen Zwischenlage, mit einem vorderen Windelrand (3), einem hinteren Windelrand (5) und zwei beide Ränder (3, 5) verbindenden Seitenrändern (7, 9), wobei jeder Seitenrand (7, 9) in seinem Mittelbereich eine mindestens angenähert trapezförmige Randaussparung aufweist, deren kürzere parallele Trapezseite innen liegt, und mit einem elastischen Abdichtungsband (15, 17), das längs jedes Seitenrandes (7, 9) in der Windel (1) mindestens an seinen beiden Enden befestigt ist, dadurch gekennzeichnet, dass jedes der befestigten Enden des Abdichtungsbandes (15, 17) im ungespannten Zustand desselben der in Endstellung untersten Querlinie (Q) näher liegt als dem benachbarten vorderen bzw. hinteren Rande (3 bzw. 5) der Windel (1) und dass die Länge jedes Abdichtungsbandes (15, 17) der Länge der zugeordneten kürzeren Trapezseite entspricht.

2. Windel nach Anspruch 1, dadurch gekennzeichnet, dass die Länge (A) jedes Abdichtungsbandes (15, 17) im ungespannten Zustand weniger als die Hälfte des Abstandes des vorderen Windelrandes (3) vom hinteren Windelrand (5) beträgt, vorzugsweise etwa 30% des genannten Abstands.

3. Vorrichtung zur Herstellung von Windeln, insbesondere Höschenwindeln, mit einer Station zum Aufbringen elastischer kontinuierlicher Abdichtstreifen auf die Aussenränder einer Bahn des Windelmaterials, bei der eine Trommel (60) an ihrem äusseren Umfang aufeinanderfolgend mit Abstand voneinander Zwischenräume (65) aufweist und eine Fördereinrichtung (66) mit Auslenkorganen (64) für eine Aussenschichtbahn für die Windel vorgesehen ist, deren Förderweg denjenigen des äusseren Umfangs der Trommel (60) in den Zwischenräumen (65) zweimal kreuzt, und die Förderwege des äusseren Umfangs und der Fördereinrichtung (66) zwischen Kreuzungsstellen mindestens auf einem Teil parallel verlaufen, und eine Zuführ- und Aufbringeinrichtung (79) für die Abdichtstreifen in den Förderweg (62) der Trägerabschnitte (63) vorgesehen ist, der eine in die Zwischenräume (65) eingreifende Trenneinrichtung (78) für die Abdichtbänder nachgeordnet ist, dadurch gekennzeichnet, dass am äusseren Umfang der Trommel (60) Trägerplatten (63) für die Aussenschichtbahn mit Abstand voneinander angeordnet sind, die Auslenkorgane (64) durch im Innern der Trommel (60) liegende Elemente parallel zum Trommelumfang führbar sind und die Fördereinrichtung (66) zwischen den Kreuzungsstellen auf einer gekrümmten Unterlage (66a) aufliegt, durch welche die Länge der Fördereinrichtung (66) verkürzt ist, und die Einrichtungen (71, 72) zur Zuführung der Abdichtstreifen in gestrecktem Zustand zur Trommel (60) vorgesehen sind.

4. Vorrichtung nach Anspruch 3, dadurch gekennzeichnet, dass vor der Trommel im Förderweg der Abdichtbänder (60) eine Bindemittelaufbringeinrichtung (77) angeordnet ist.

5. Vorrichtung nach mindestens einem der Ansprüche 3 bis 4, dadurch gekennzeichnet, dass die Trenneinrichtung (78) ein rotierendes Messer ist.

6. Vorrichtung nach mindestens einem der Ansprüche 3 bis 5, dadurch gekennzeichnet, dass nach der Trenneinrichtung (78) Einrichtungen (30, 44, 32, 33) zum Zuführen von einer saugfähigen Zwischenlage und einer Innenschicht für die Windel zum Verbinden derselben mit der Bahn der Aussenschicht vorgesehen sind.

7. Vorrichtung nach Anspruch 6, dadurch gekennzeichnet, dass eine Einrichtung zum portionsweisen Ablegen der Zwischenlage vorgesehen ist.

8. Vorrichtung nach Anspruch 6 oder 7, dadurch gekennzeichnet, dass vor der Zuführeinrichtung (32, 33) für die Innenschicht und die Zwischenlage eine Bindemittelauftrageinrichtung (44) auf die Bahn der Aussenschicht vorgesehen ist.

9. Vorrichtung nach mindestens einem der Ansprüche 3 bis 8, dadurch gekennzeichnet, dass die Fördereinrichtung zwei Förderketten (66) aufweist, zwischen denen Stangen (64) als die Auslenkorgane angeordnet sind und die zur Verkürzung der Länge der Fördereinrichtung auf einer gekrümmten Führung (66a) als Unterlage im Innern der Trommel (60) aufliegen.

10. Vorrichtung nach Anspruch 9, dadurch gekennzeichnet, dass die Trägerplatten (63) mit einem Abstand voneinander angeordnet sind, dass die Stangen (64) mit kleinem Spiel durch die Zwischenräume (65) hindurchführbar sind.

## Claims

1. Nappies, particularly panty nappies, with an outer layer impermeable to liquid, an inner layer permeable to liquid and an absorbent intermediate layer, with a front nappy edge (3), a rear nappy edge (5) and two side edges (7, 9) connecting both edges (3, 5), whereby each side edge (7, 9) has in its central region one at least almost trapezoidal edge recess, the short parallel trapezoidal side of which lies in the interior and has an elastic sealing strip (15, 17) which is attached at least at both its ends along each side edge (7, 9) in the nappy (1), characterised in that each of the attached ends of the sealing strip (15, 17) in an unstretched state of the same is positioned closer to the lowest transverse line (Q) in a final position than to the adjacent front or rear edge (3 or 5) of the nappy (1), and that the length of each sealing strip (15, 17) corresponds to the length of the corresponding shorter trapezoidal side.

2. Nappies according to claim 1, characterised in that the length (A) of each sealing strip (15, 17) in an unstretched state is less than half the spacing of the front nappy edge (3) from the rear nappy edge (5), preferably about 30% of the said spacing.

3. A device for the production of nappies, particularly panty nappies, with a station for applying continuous elastic sealing strips on the outer edges of a length of nappy material, in which a drum (60) has successive intermediate spaces (65) on its outer periphery at a spacing from each other and a conveying device (66) with deflecting members (64) for a path of an outer layer of the nappy is provided, the conveying route of which twice crosses that of the outer periphery of the drum (60) in the intermediate spaces (65), and the conveying routes of the outer periphery and the conveying device (66) between crossing points run parallel at least on one section, and a supply and application device (79) is provided for the sealing strips in the conveying route (62) of the carrier sections (63), which is connected downstream of a separating device (78) for the sealing strips which engages in the intermediate spaces (65), characterised in that on the outer periphery of the drum (60), carrier plates (63) are arranged for the path of the outer layer at a spacing from each other, the deflecting members (64) can be guided parallel to the drum periphery by elements lying in the interior of the drum (60), and the conveying device (66) is positioned between the crossing points on a curved base (66a), by which the length of the conveying device (66) is shortened, and the devices (71, 72) for supplying the sealing strips in a stretched state to the drum (60) are provided.

4. A device according to claim 3, characterised in that a binder application device (77) is arranged upstream of the drum in the conveying route of the sealing strips (60).

5. A device according to at least one of claims 3 to 4, characterised in that the separating device (78) is a rotating cutter.

6. A device according to at least one of claims 3 to 5, characterised in that downstream of the separating device (78), devices (30, 44, 32, 33) are provided for supplying an absorbent intermediate layer and an inner layer for the nappy and for joining the same with the path of the outer layer.

7. A device according to claim 6, characterised in that a device is provided for the portion-wise laying of the intermediate layer.

8. A device according to claim 6 or 7, characterised in that a binder application device (44) is provided on the path of the outer layer upstream of the supply device (32, 33) for the inner layer and the intermediate layer.

9. A device according to at least one of claims 3 to 8, characterised in that the conveying device has two conveyor chains (66), between which rods (64) are arranged as the deflection organs and which for shortening the length of the conveying device are positioned on a curved guide (66a) as a base in the interior of the drum (60).

10. A device according to claim 9, characterised in that the carrier plates (63) are arranged at a spacing from each other, and that the rods (64) can be guided through the intermediate spaces (65) with a small amount of play.

## Revendications

1. Couche, en particulier couche-culotte, comprenant un revêtement externe imperméable aux liquides, un revêtement interne perméable aux liquides et une couche intermédiaire absorbante, un bord antérieur (3), un bord postérieur (5) et deux bords latéraux (7, 9) reliant ces deux bords (3, 5), chaque bord latéral (7, 9) présentant, dans sa région centrale, un évidement marginal au moins approximativement trapézoïdal dont le côté parallèle le plus court du trapèze est situé à l'intérieur, ainsi qu'un ruban élastique d'étanchéité (15, 17) fixé, dans la couche (1), au moins par ses deux extrémités le long de chaque bord latéral (7, 9), caractérisée par le fait que chacune des extrémités assujetties du ruban d'étanchéité (15, 17) se trouve, en condition détendue de ce ruban, plus près de la ligne transversale (Q) située le plus bas dans la position extrême, que du bord voisin respectivement antérieur ou postérieur (3; 5) de la couche (1); et par le fait que la longueur de chaque ruban d'étanchéité (15, 17) correspond à la longueur du côté associé le plus court du trapèze.

2. Couche selon la revendication 1, caractérisée par le fait que la longueur (A) de chaque ruban d'étanchéité (15, 17) représente, en condition détendue, moins de la moitié de la distance entre le bord antérieur (3) de la couche et le bord postérieur (5) de cette couche, de préférence environ 30% de la distance précitée.

3. Dispositif pour fabriquer des couches, en particulier des couches-culottes, comprenant un poste pour déposer des bandes élastiques ininterrompues d'étanchéité sur les bords externes d'un lé du matériau constituant la couche, dans lequel un tambour (60) comporte sur son pourtour externe des espaces intercalaires (65) se succédant à distance les uns des autres, et dans lequel il est prévu un dispositif de convoyage (66) doté d'organes (64) de renvoi d'un lé du revêtement externe de la couche, la trajectoire d'acheminement de ce dispositif croisant deux fois celle du pourtour externe du tambour (60) dans les espaces intercalaires (65), les trajectoires d'acheminement du pourtour externe et du dispositif de convoyage (66) s'étendant parallèlement au moins sur un tronçon entre des zones de croisement, et dans lequel il est prévu un dispositif (79) qui délivre et dépose les bandes d'étanchéité dans la trajectoire d'acheminement (62) des tronçons de support (63), et en aval duquel est installé un dispositif (78) séparant les rubans d'étanchéité et s'engageant dans les espaces intercalaires (65), caractérisé par le fait que des plaques (63) de support du lé de revêtement externe sont disposées à distance les unes des autres sur le pourtour externe du tambour (60); par le fait que les organes de renvoi (64) peuvent être guidés parallèlement au pourtour du tambour par l'intermédiaire d'éléments situés à l'intérieur de ce tambour (60); par le fait que le dispositif de convoyage (66) repose, entre les zones de croisement, sur une structure sous-jacente curviligne (66a) par laquelle la longueur de ce dispositif de convoyage (66) est raccourcie; et par le fait que les dispositifs (71, 72) pour délivrer les bandes d'étanchéité sont prévus en condition déployée par rapport au tambour (60).

4. Dispositif selon la revendication 3, caractérisé par le fait qu'un dispositif (77) pour déposer un liant se trouve avant le tambour dans la trajectoire d'acheminement des rubans d'étanchéité (60).

5. Dispositif selon au moins l'une des revendications 3 à 4, caractérisé par le fait que le dispositif de séparation (78) consiste en un couteau rotatif.

6. Dispositif selon au moins l'une des revendications 3 à 5, caractérisé par le fait que des dispositifs (30, 44, 32, 33) sont prévus, après le dispositif de séparation (78), pour délivrer une couche intermédiaire absorbante et un revêtement interne de la couche, de façon à relier ces derniers au lé du revêtement externe.

7. Dispositif selon la revendication 6, caractérisé par le fait qu'il est prévu un dispositif pour déposer la couche intermédiaire par portions.

8. Dispositif selon la revendication 6 ou 7, caractérisé par le fait qu'un dispositif (44) de dépôt d'un liant sur le lé du revêtement externe est prévu avant le dispositif (32, 33) délivrant le revêtement interne et la couche intermédiaire.

9. Dispositif selon au moins l'une des revendications 3 à 8, caractérisé par le fait que le dispositif de convoyage comprend deux chaînes convoyeuses (66) entre lesquelles sont intercalées des tiges (64) constituant les organes de renvoi et qui, pour raccourcir la longueur du dispositif de convoyage, portent sur un élément de guidage curviligne (66a) en tant que structure sousjacente à l'intérieur du tambour (60).

10. Dispositif selon la revendication 9, caractérisé par le fait que les plaques de support (63) sont mutuellement distantes d'un espacement tel que les tiges (64) puissent traverser de part en part les espaces intercalaires (65) avec un faible jeu.

Fig. 1

Fig. 1a

Fig. 2

Fig. 3

0 066 545

Fig. 4

Fig. 5

Fig. 6